# EUROPEAN PATENT SPECIFICATION

(11) **EP 1 572 179 B1**
(45) Date of publication and mention of the grant of the patent: **28.02.2007**
(21) Application number: 03795825.3
(22) Date of filing: 18.11.2003
(51) Int. Cl.: A61K 31/155, A61K 31/5415, A61K 45/06, A61P 29/00, A61P 19/02, A61P 11/06, A61P 11/00, A61P 17/00, A61P 17/06, A61P 35/04, A61P 1/04, A61P 1/00, A61P 25/00, A61P 9/10

(54) **PHARMACEUTICAL COMPOSITION COMPRISING A LTB4 ANTAGONIST AND A COX-2 INHIBITOR OR A COMBINED COX1/2 INHIBITOR**
PHARMAZEUTISCHE ZUSAMMENSETZUNG ENTHALTEND EINEN LTB4-HEMMER SOWIE EINEN COX-2-HEMMER ODER EINEN KOMBINIERTEN COX1/2-HEMMER
COMPOSITION PHARMACEUTIQUE CONTENANT UN ANTAGONISTE DE LTB4 ET UN INHIBITEUR DE COX-2 OU UN INHIBITEUR A LA FOIS DE COX-1 ET COX-2

(30) Priority: 26.11.2002 EP 02026223
(43) Date of publication of application: 14.09.2005
(73) Proprietor: Boehringer Ingelheim Pharma GmbH & Co. KG, 55216 Ingelheim (DE)
(72) Inventor: BIRKE, Franz, 55218 Ingelheim (DE); JENNEWEIN, Hans, Michael, 65193 Wiesbaden (DE)
(86) International application number: PCT/EP2003/012874
(87) International publication number: WO 2004/047824

(56) References cited:
- WO-A-97/21670
- WO-A-98/11062
- WO-A-03/007922
- US-A1- 2002 107 276
- US-A1- 2002 119 193
- US-B1- 6 172 096
- BIRKE, F. W. ET AL: "In vitro and in vivo pharmacological characterization of BIIL 284, a nove and potent leukotriene B4 receptor antagonist" JOURNAL OF PHARMACOLOGY AND EXPERIMENTAL THERAPEUTICS (2001), 297(1), 458-466 , XP001079480
- BARNES P.J.: "Future advances in COPD therapy." RESPIRATION, (2001) 68/5 (441-448). , XP001084651

## Description

### BACKGROUND OF THE INVENTION

### 1. TECHNICAL FIELD

The invention relates to a pharmaceutical composition comprising an orally available, LTB₄ antagonist of formula (I), or a tautomer, a pharmaceutically acceptable salt or solvate thereof **(1)** and meloxicam or a pharmaceutically acceptable salt or solvate thereof **(2),** and a pharmaceutically acceptable carrier or excipient, and optionally one or more other therapeutic ingredients.

### 2. BACKGROUND INFORMATION

The US patent US 6,172,096 discloses a method of reducing recipient acute or chronic rejection of transplanted cells or organs, and for treatment of autoimmune diseases, hypersensitivity reactions of the acute or delayed type, allergic disorders, granulomas, meningitis, and septic shock by administering a cyclooxygenase-2 inhibitor and a leukotriene B₄ (LTB₄) receptor antagonist.

The US patent US 2002/107276 discloses a combination of a cyclooxygenase-2 inhibitor and an leukotriene B4 receptor antagonist for the treatment of inflammation and inflammation-related disorders.

LTB₄ antagonists which contain a hydroxy and benzamidine group are compounds with pharmacologically valuable properties. Such LTB₄ antagonists may provide great therapeutic benefit, for example, in the treatment of arthritis, asthma, chronic obstructive lung diseases, psoriasis, ulcerative colitis, Alzheimer's disease, shock, reperfusion damage/ischaemia, cystic fibrosis, atherosclerosis and multiple sclerosis.

Such compounds are known e.g. from International Patent Applications WO 96/02497, WO 97/21670, WO 98/11062, WO 98/11119, WO O1/25186 and PCT/EP01/00262 .

However, none of these prior art references indicates that the combination of a cyclooxygenase-2 inhibitor and a LTB₄ antagonist of formula (I) will show a synergistic effect, in particular for the treatment of rheumatic arthtitis.

It has been demonstrated that arachidonic acid produced a dermal inflammation when applied topically (Carter et al, 5-Lipoxygenase inhibitory activity of zileuton. J Pharmacol Exp Ther 256 929-937 (1990)). This inflammation is rich in neutrophils and consequently myeloperoxidase (MPO), a neutrophil marker enzyme, can be used as a quantitative index for cell infiltration. The mouse ear is especially suited to serve as a model of dermal inflammation induced by various agents like arachidonic acid which is known to be metabolized to several inflammatory mediators i.e. prostaglandines and LTB₄. Accordingly neither an LTB₄ antagonist nor an NSAID alone are supposed to fully counteract this kind of inflammation. Therefore this model seems to be useful to test the efficacy of a NSAID-LTB₄ antagonist combination.

It has now be found surprisingly that a pharmaceutical formulation comprising the cyclooxygenase-2 inhibitor meloxicam and a LTB₄ antagonist of formula (I) shows a synergistic effect, in particular for the treatment of rheumatic arthtitis.

### BRIEF SUMMARY OF THE INVENTION

The invention relates to a pharmaceutical composition comprising a LTB₄ antagonist having a hydroxy and a benzamidine group of formula (I) 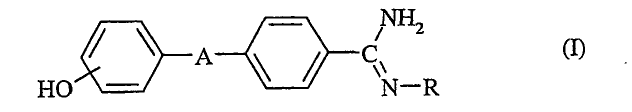 wherein
R represents a hydrogen atom or a group of formula -CO₂-R', in which R' represents a C₁₋₆ alkyl, an optionally substituted phenyl group or an optionally substituted benzyl group, wherein the optional substituents are selected from halogen atoms, C₁₋₆ alkyl, C₁₋₆ alkoxy, cyano, nitro; C₁₋₆ haloalkyl and C₁₋₆ haloalkoxy groups, and
A is a group selected from the formula (A1): 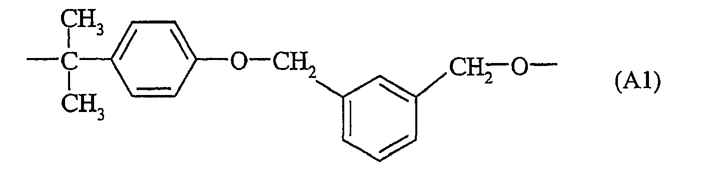
or a tautomer, a pharmaceutically acceptable salt or solvate thereof (**1**), and
the cyclooxygenase-2 inhibitor or combined cox1/cox2 inhibitor meloxicam or a pharmaceutically acceptable salt or solvate thereof (**2**), and a pharmaceutically acceptable carrier or excipient, and optionally one or more other therapeutic ingredients.

R preferably represents H or -CO₂-C₂H₅.

Another aspect of the present invention is the use of effective amounts of a LTB₄ antagonist having a hydroxy and a benzamidine group of formula (I) **(1)** and the cyclooxygenase-2 or combined cox1/cox2 inhibitor meloxicam **(2)** to a patient in need thereof in a combined form, or separately, or separately and sequentially, wherein the sequential administration is to be close in time or remote in time, for the manufacture of a medicament for the prevention or treatment of a disease or disorder selected from the group consisting of arthritis, including rheumatoid arthritis, spondyloarthropathies, gouty arthritis, osteoarthritis, systemic lupus erythematosus and juvenile arthritis, asthma, hay fever, atopic dermatitis,rhinitis, bronchitis, COPD and cystic fibrosis, psoriasis, sclerodermia, morbus bechterew, sarcoidosis, tumor metastasis,morbus crohn, colitis ulcerosa, IBD, multiple sclerosis, arteriosclerosis, arteritis, myocardial infarction, stroke, coronary heart disease.

Furthermore, the invention relates to the use of a LTB₄ antagonist having a hydroxy and a benzamidine group of formula (I) **(1)** and meloxicam **(2)** in a combined form, or separately, or separately and sequentially, wherein the sequential administration is to be close in time or remote in time, for the manufacture of a medicamentation for the prevention or treatment of disease or disorder selected from the group consisting of arthritis, including rheumatoid arthritis, spondyloarthropathies, gouty arthritis, osteoarthritis, systemic lupus erythematosus and juvenile arthritis, asthma, bronchitis, COPD and cystic fibrosis.

Finally, the invention relates to pharmaceutical kit comprising at least two separate unit dosage forms (A) and (B):
(A) one of which comprises a composition containing a LTB₄ antagonist having a hydroxy and a benzamidine group of formula (I), a tautomer thereof or a pharmaceutically acceptable salt thereof **(1)**, and optionally a pharmaceutically acceptable carrier;
(B) one of which comprises a composition containing the cyclooxygenase-2 inhibitor or combined cox1/ 2 inhibitor meloxicam **(2)**, and optionally a pharmaceutically acceptable carrier.

### DETAILED DESCRIPTION OF THE INVENTION

The term "LTB₄ antagonists of formula (I) embraces compounds which selectively inhibit the leukotriene B₄ receptor.

The term "C₁₋₆ alkyl" embraces straight chained and branched alkyl groups having 1 to 6 carbon atoms such as methyl, ethyl *n*-propyl, *i*-propyl, *n*-butyl, 2-butyl, *n*-pentyl and *n*-hexyl.

Preferred is a pharmaceutical composition, wherein the active principle essentially consists of a compound of formula (I), in particular formula (IA) 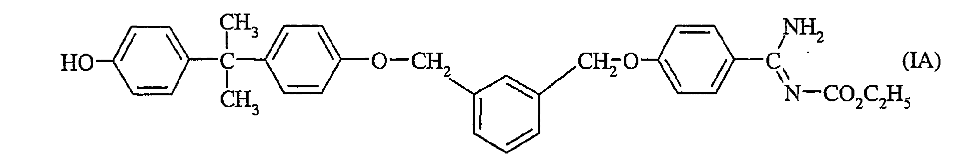 **(1)** and the cyclooxygenase-2 inhibitor or combined cox1/cox2 inhibitor meloxicam of formula 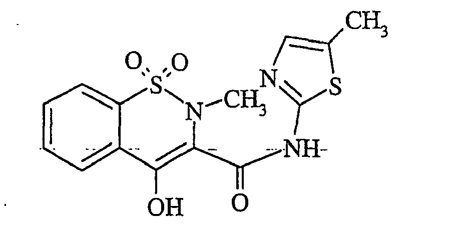 or a pharmaceutically acceptable salt thereof.

The phrase "combination therapy" (or "co-therapy"), in defining use of the cyclooxygenase-2 inhibitor agent meloxicam and a leukotriene B₄ receptor antagonist agent of formula (I), is intended to embrace administration of each agent in a sequential manner in a regimen that will provide beneficial effects of the drug combination. The phrase also is intended to embrace co-administration of these agents in a substantially simultaneous manner, such as in a single capsule having a fixed ratio of these active agents or in multiple, separate capsules for each agent.

The phrase "therapeutically-effective" is intended to qualify the amount of each agent for use in the combination therapy which will achieve the goal of improvement in severity and the frequency of disease incidence over treatment of each agent by itself, while avoiding adverse side effects typically associated with alternative therapies.

The active substance of formula I may be present in the formulation according to the invention in the form of a physiologically acceptable acid addition salt. By physiologically acceptable acid addition salts are meant, according to the invention, pharmaceutically acceptable salts which are selected from the salts of hydrochloric acid, hydrobromic acid, sulphuric acid, phosphoric acid, methanesulphonic acid, acetic acid, fumaric acid, succinic acid, lactic acid, citric acid, tartaric acid and maleic acid. Mixtures of the above acids may also be used to prepare the salts. According to the invention, the preferred salts of formula I are selected from among the hydrochloride, hydrobromide, sulphate, phosphate, fumarate and methanesulphonate. The salts selected from among the hydrochloride, hydrobromide and fumarate are particularly preferred. The active substance may optionally be in the form of a hydrate. Preferably, according to the invention, the compound of formula I is added to the tablet in the form of the free base and in the anhydrous form.

The pharmaceutical formulation according to the present invention is as a rule suitable for oral, intravascular, intraperitoneal, subcutaneous, intramuscular or topical administration, in particular oral administration.

The present invention preferably relates to a tablet containing a compound of formula I and meloxicam which contains at least one pharmacologically acceptable excipient, and optionally at least one wetting agent.

The term "wetting agent" as used hereinbefore and hereinafter denotes an excipient which lowers the surface tension of water or other liquids so that they can penetrate into the surfaces of the tablets according to the invention and soak through them, displacing the air, thereby wetting them. The substances used as wetting agents are usually interface-active surfactants. These surfactants are amphiphilic (bifunctional) compounds with at least one hydrophobic and one hydrophilic part of the molecule. The hydrophobic group is usually a hydrocarbon chain, if possible a straight chain, with eight to 22 carbon atoms. Particular surfactants may also have (dimethyl)-siloxane chains or perfluorinated hydrocarbon chains as the hydrophobic part of the molecule. The hydrophilic group is either a negatively or positively charged (hydratable) or a neutral polar head group. Of the surfactants, anionic surfactants, particularly the long-chain alkylsulphates, especially sodium laurylsulphate and alkylbenzenesulphonates are preferred.

Within the scope of the present invention carbohydrates such as lactose or mannose, particularly finely divided lactose or sugar alcohols such as mannitol, sorbitol or xylitol, particularly mannitol, are of particular importance as excipients. These excipients have proved particularly advantageous in the formulation according to the invention. In a preferred aspect, therefore, the present invention relates to a tablet according to claim 1, containing at least one compound of formula I, which contains, in addition to the active substance and the wetting agent, lactose, particularly finely divided lactose, more preferably lactose monohydrate or mannitol as excipient.

The tablet according to the invention may also contain compounds capable of acting as binders.

The term "binder" used hereinbefore and hereinafter denotes excipients which are suitable for binding other components to one another. Preferred binders according to the invention are selected from among:
powdered cellulose, microcrystalline cellulose, sorbitol, starch, polyvinylpyrrolidone (povidone), copolymers of vinylpyrrolidone with other vinyl derivatives (copovidone), cellulose derivatives, particularly methylhydroxypropylcellulose, e.g. Methocel A 15.LV, and mixtures of these
compounds. The preferred binders are-powdered cellulose, particularly microcrystalline cellulose and/or copovidone. Most preferred is a mixture of microcrystalline cellulose and a copolymer of vinylpyrrolidone and vinyl acetate, namely copovidone VA 64, the ratio of vinylpyrrolidone and
vinyl acetate being about 3:2 (m/m). As a rule the tablet according to the invention has a weight ratio of microcrystalline cellulose to copovidone VA 64 of 20:1 1 to 1:1, preferably 15:1 1 to 2:1, particularly about 10:1 to 3:1. Thanks to this particularly preferred binder combination of microcrystalline cellulose and copovidone, tablets are obtained having a high bioavailability of the compounds of formula I.

The tablet according to the invention may also contain disintegrants in addition to the above mentioned ingredients. Within the scope of the present invention these disintegrants may optionally also be known as breakdown agents. These are preferably selected, according to the invention, from among sodium starch glycolate, crosslinked polyvinylpyrrolidone (crospovidone), croscarmellose sodium salt (sodium salt of cellulose carboxymethyl ether, crosslinked), sodium-carboxymethylcellulose, dried maize starch and mixtures thereof. Within the scope of the present invention it is particularly preferred to use sodium starch glycolate, crospovidone and, preferably, the sodium salt of crospovidone or croscarmellose.

The tablet according to the invention may also contain flow agents or flow regulators and also lubricants, as additional ingredients. These include, within the scope of the present invention, for example, silicon dioxide, talc, stearic acid, sodium stearylfumarate, magnesium stearate and glycerol tribehenate. According to the invention magnesium stearate is preferably used.

In addition, the tablet according to the invention may contain one or more synthetic or natural, pharmaceutically acceptable dyes or colourings, preferably indigo carmine. If the abovementioned colourings are used the amount by weight thereof based on the total mass of the tablet according to the invention is 0.01 to 0.5 wt.%.

The active ingredients **(1)** and **(2)** are as a rule applied in a ratio, in which the resulting combination exhibits a synergistic effect. The term "synergistic effect" as used herein relates to an effect, which is higher than one could expect from the additive effects of each single active ingredient.

Accordingly, the pharmaceutical formulation according to the present invention exhibits as a rule **(1)** and **(2)** in synergistically effective amounts of, preferably a weight ratio of **(1)** to **(2),** which ranges from 50 : 1 to 1 : 300, preferably from 8:1 to 1:80, in particular 1:1 to 1:40, most preferably 1:5 to 1:30.

The pharmaceutical formulation according to the present invention are preferably administered in a single application dose containing 1 to 10,000 milligrams, preferably 5 to 1000 mg of the combined active ingredients **(1)** and **(2).** Most preferred is a formulation comprising about 10 mg meloxicam and up to 300 mg of formula IA.

The Examples that follow serve to illustrate some formulations according to the invention. They are intended solely as possible procedures described by way of example.

### Example 1

| **Ingredients** | mg/tablet |
|---|---|
| (01) compound IA, jet-ground | 1,000 |
| (02) meloxicam | 10,000 |
| (03) microcrystalline cellulose | 15,000 |
| (04) mannitol | 52,250 |
| (05) croscarmellose sodium | 1,500 |
| (06) sodium laurylsulphate | 0,050 |
| (07) indigo carmine (11-14 %) | 0,075 |
| (08) magnesium stearate | 1,125 |
| | 81,000 |

The direct compression comprises producing a premix of ingredients (01), (02), (06), (07) and some of (04) with an intensive mixer. The premix is screened and mixed with ingredients (03), (05) and the remainder of (04) in a gravity mixer. After the mixture has been screened again, ingredient (08) is added.

### Example 2

### 2.1 Animals

Female albino mice (Han:NMRI) obtained from Interfauna and weighing about 20-25 g were used. The animals were provided with standardised pellet diet (Altromin 8013) and had tap water freely available. The animals were accommodated in a climatized room with a 12 hours light/dark cycle and kept in groups.

### 2.2 Chemical substances

### Compound of formula IA:

Carbamic acid, [[4-[[3-[[4-[1-(4-hydroxyphenyl)-1-methylethyl]-phenoxy]methyl]phenyl]methoxy]phenyl]iminomethyl]-, ethyl ester was prepared as described in US 6,417,382.

***Meloxicam*** was provided by Boehringer Ingelheim Pharma KG

***Arachidonic acid*** was purchased from Sigma (A9798) and dissolved 1:10 in acetone.

### 2.3 Study Design

The compound of formula IA and meloxicam were administered orally (0.2 ml / 10 g bw) 30 min. before arachidonic acid challenge. Meloxicam was given twice: one dose 16 hours and the second dose 30 minutes before challenge. For every day there was a concurrent control. Then number of animals per group was 7. The study compounds were suspended in 1% methylcellulose (Tylose MH 300, Fluka,CH-9470 Buchs). The experiment was run in five groups. Details are given in Table 1. Every group contained one control, two doses of meloxicam, two doses of (IA), and one dose of the combination of the two compounds.

**Table 1**

| Treatment | N | Dose m/kg p.o.) Group 1 | Dose m/kg p.o.) Group 2 | Dose m/kg p.o.) Group 3 | Dose m/kg p.o.) Group 4 | Dose m/kg p.o.) Group 5 |
|---|---|---|---|---|---|---|
| Control (Tylose) | 7 | | | | | |
| Meloxicam | 7 | 1 | 2 | 4 | 8 | 16 |
| Meloxicam | 7 | 2 | 4 | 8 | 16 | 32 |
| (IA) | 7 | 0.05 | 0.1 | 0.2 | 0.4 | 0.8 |
| (IA) | 7 | 0.1 | 0.2 | 0.4 | 0.8 | 1.6 |
| Meloxicam plus (IA) | | 1 | 2 | 4 | 8 | 16 |
| | 7 | 0.05 | 0.1 | 0.2 | 0.4 | 0.8 |

### 2.4 Experimental Procedure

Mice were lightly anesthetized by ether and 1 mg arachidonic acid (10 µl) was applied to each side of the left ear. The right ear remained untreated, acetone alone did not cause any late response. The animals were sacrificed by ether 6 hours later, and a biopsy (diameter 8 mm) was punched out from both ears to assess an increase of neutrophils in the left ear compared with the right ear. Tissue samples were homogenized in 1 ml 0.5% HTAB (Hexadecyl-trimethyl-ammonium-bromide; Sigma H-5882; solved in 0.05 M phosphate buffer, pH 6.0) using a tissue homogenizer (IKA-Ultraturrax T5; Janke & Kunkel, Staufen/Breisgau) at 30000 RPM for 15 seconds. After centrifugation (16000 G, 5 min) the supernatants were frozen until processing for myeloperoxidase (MPO). Determination in the supernatants for MPO, a neutrophil marker enzyme, served as a quantitative index for the neutrophil accumulation.

MPO was determined spectrophotometrically at 450 nm using a micro plate version of the method of Bradley (1982) and a micro plate reader (Vₘₐₓ; Molecular Devices, Palo Alto) suitable for kinetic measurements and expressed as mean optical density per minute.

### 2.5 Statistical Evaluation

For each individual the response myeloperoxidase activity (MPO) in optical density per minute (oD/min) was measured.

Based on these results the following statistical analyses were done:
- Comparison of control groups
- Comparison of Control vs. Treatments separately for each group
- Comparison of high vs. low dose for both treatments separately for each group
- Comparison of combined treatment vs. high doses separately for each group
- Comparison of combined treatment vs. other treatments in group 5
For the comparisons the nonparametric statistical methods Kruskal-Wallis-One-Way-ANOVA and the Wilcoxon-Two-Sample-Test (2sided) have been used. For the first and last comparisons also Bonferroni-Holm adjusted p-values have been calculated.

The calculations and statistical analysis were done with the NPAR1WAY procedure of the SAS software program (SAS Institute Inc., Cary, North Carolina) version 8.2. (IA) and meloxicam both inhibited arachidonic acid induced ear inflammation in mice. Details of statistical analysis are shown in tables 2 to 4.

**Table 2 Difference between treatment and control**

| **Group** | **Treatment** Compound / dose [mg/kg] | **p-value** | **p-value (adj)** |
|---|---|---|---|
| 1 | Meloxicam / 1 | 1.0000 | n.s. |
| | Meloxicam / 2 | 0.2502 | n.s. |
| | (IA) / 0.05 | 0.7983 | n.s. |
| | (IA) / 0. | 0.7491 | n.s. |
| | Meloxicam / 1+(IA) / 0.05 | 0.8983 | n.s. |
| 2 | Meloxicam / 2 | 0.0152 | 0.0304 |
| | Meloxicam / 4 | 0.1252 | 0.1252 |
| | (IA) / 0.1 | 0.0060 | 0.0240 |
| | (IA) / 0.2 | 0.0073 | 0.0219 |
| | Meloxicam / 2 + (IA) /0.1 | 0.0022 | 0.0110 |
| 3 | Meloxicam / 4 | 0.0736 | 0.0736 |
| | Meloxicam / 8 | 0.0049 | 0.0147 |
| | (IA) / 0.2 | 0.0073 | 0.0146 |
| | (IA) / 0.4 | 0.0033 | 0.0165 |
| | Meloxicam / 4 + (IA) / 0.2 | 0.0033 | 0.0165 |
| 4 | Meloxicam/ 8 | 0.3067 | n.s. |
| | Meloxicam / 16 | 0.7015 | n.s. |
| | (IA) / 0.4 | 0.0553 | 0.1659 |
| | (IA) / 0.8 | 0.0106 | 0.0424 |
| | Meloxicam /8 + (IA) / 0.4 | 0.0022 | 0.0110 |
| 5 | Meloxicam /16 | 0.1599 | n.s. |
| | Meloxicam / 32 | 0.2246 | n.s. |
| | (IA)/0.8 | 0.0049 | 0.0196 |
| | (IA) / 1.6 | 0.0072 | 0.0216 |
| | Meloxicam /16 + (IA) / 0.8 | 0.0017 | 0.0085 |

**Table 3**

| Comparisons High/Low Treatment and High Treatment vs Combination | | | |
|---|---|---|---|
| **Group** | **Low Dose [mg/kg]** | **High Dose [mg/kg]** | **p-value** |
| 1 | Meloxicam/1 | Meloxicam /2 | 0.3067 |
| | (IA)/0.05 | (IA)/0.1 | 0.8983 |
| 2 | Meloxicam / 2 | Meloxicam / 4 | 0.7983 |
| | (IA) / 0.1 | (IA) / 0.2 | 0.7983 |
| 3 | Meloxicam / 4 | Meloxicam / 8 | 0.0474 |
| | (IA) / 0.2 | (IA) / 0.4 | 0.5229 |
| 4 | Meloxicam / 8 | Meloxicam / 16 | 0.7983 |
| | (IA)/0.4 | (IA)/0.8. | 0.9490 |
| 5 | Meloxicam/16 | Meloxicam/32 | 0.4320 |
| | (IA)/0.8 | (IA)/1.6 | 0.7012 |
| | | | |

| **Group** | **High Dose [mg/kg]** | **Combination[mg/kg]** | **p-value** |
|---|---|---|---|
| 1 | Meloxicam / 2 | Meloxicam / 1 + (IA)/0.05 | 0.1792 |
| | (IA) / 0.1 | Meloxicam / 1 + (IA) / 0.05 | 0.8983 |
| 2 | Meloxicam / 4 | Meloxicam / 2 + (IA) / 0.1 | 0.0033 |
| | (IA) / 0.2 | Meloxicam / 2 + (IA) / 0.1 | 0.0348 |
| 3 | Meloxicam/8 | Meloxicam /4 + (IA) / 0.2 | 1.0000 |
| | (IA) / 0.4 | Meloxicam / 4 + (IA)/0.2 | 0.3067 |
| 4 | Meloxicam /16 | Meloxicam / 8 + (IA) / 0.4 | 0.0033 |
| | (IA)/0.8 | Meloxicam /8 +(IA) / 0.4 | 0.0215 |
| 5 | Meloxicam / 32 | Meloxicam /16 + (IA) / 0.8 | 0.0026 |
| | (IA) /1.6 | Meloxicam /16 + (IA) /0.8 | 0.0086 |

**Table 4**

| Comparisons Combination vs Others for Group 5 | | |
|---|---|---|
| **Comparison vs combined (Group5)** | **p-value** | **p-value (adj)** |
| Control | 0.0017 | 0.0085 |
| Meloxicam / 16 | 0.0032 | 0.0096 |
| Meloxicam / 32 | 0.0026 | 0.0104 |
| (IA) / 0,8 | 0.0090 | 0.0090 |
| (IA) /1,6 | 0.0086 | 0.0172 |

The arachidonic acid induced mouse ear inflammation test is indicative for pathological processes where neutrophils are involved (see also introduction). It shed light especially on the chemoattractant part of the arachidonic acid cascade induced neutrophil activation. The results indicate that (IA) and Meloxicam are effective orally and consequently may target important parts of this inflammation.

With respect to the combination of both compounds, there are two criteria which must be fulfilled to prove a super-additive efficacy (potentiation).
i.) the maximal achievable effect of the combination must be bigger than the maximal achievable effect of the single compound.
ii.) the effect of the combination should be bigger than the effect which can be expected from the dose response curve of the single compounds

The first criterion is experimentally tested by using supra-maximum doses of the single compounds and compare these with the achievable maximal effect of the combination. This may be difficult when the compounds under investigation produce a 100% inhibition itself. Therefore the model of arachidonic acid induced mouse ear inflammation was chosen, because NSAIDs and LTB₄ antagonists demonstrate very shallow dose response curves and a 100% inhibitory effect is hardly possible. As shown in the present experiments of group 5 the supra-maximum doses of meloxicam (16 and 32 mg/kg p.o.) and (IA) (0.8 and 1.6 mg/kg p.o.) achieved maximum inhibitory effects of 37% and 66% respectively, whereas the combination (meloxicam 16 mg/kg p.o., (IA) 0.8mg/kg p.o.) achieved a maximum inhibition of 96%. This difference was statistically significant and consequently proves a super-additive effect according to criterion i.).

The second criterion can be tested by doubling the doses of the single compounds and compare the effect of the higher doses of the single compounds with the effect of the combination of the lower doses of the single compounds. In group 1 all doses were too low to achieve any effect and consequently the results of this experiment cannot be used either to accept or reject the hypothesis of potentiation. The same holds true for the experiments in group 3. Although the combination achieved the highest inhibition, the difference to the inhibition reached with the highest doses of the single compounds were not statistically significant, because the single compounds alone caused already high inhibition values. However the experiments performed in group 2, 4, and 5 clearly show that the combination was significantly more effective than the higher doses of the single compounds thus proving a super-additive efficacy according to criterion i.i.

It is concluded that the combination of the non steroidal anti inflammatory drug meloxicam with the LTB₄ antagonist (IA) strongly inhibits arachidonic acid induced mouse ear inflammation after oral administration in an super-additive way.

## Claims

1. A pharmaceutical composition comprising a LTB₄ antagonist of formula (I) 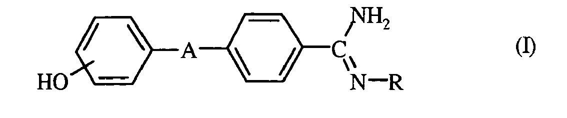 wherein R represents a hydrogen atom or a group of formula -CO₂-R', in which R' represents a C₁₋₆ alkyl, an optionally substituted phenyl or an optionally substituted benzyl group, wherein the optional substituents are selected from halogen atoms, C₁₋₆ alkoxy, Cyano, nitro; C₁₋₆ haloalkyl and C₁₋₆haloalkoxy groups, and A is a group selected from the formula (A1): 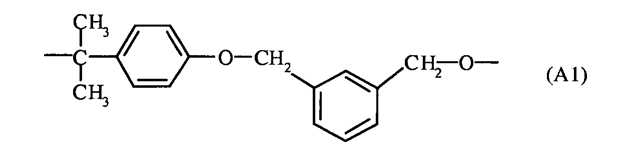 or a tautomer, a pharmaceutically acceptable salt or solvate thereof **(1)** and meloxicam of formula 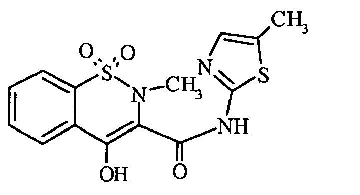 or a pharmaceutically acceptable salt thereof **(2)**, and a pharmaceutically acceptable carrier or excipient.

2. A pharmaceutical composition according to claim 1 consisting essentially of the compound of formula (IA) 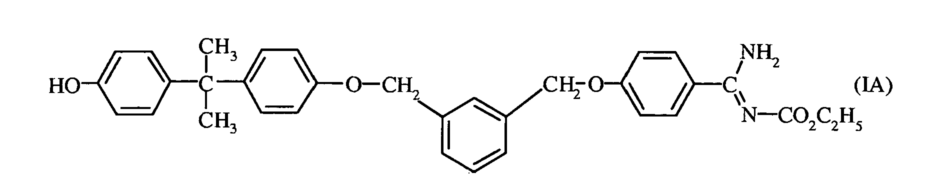 **(1)** and meloxicam of formula 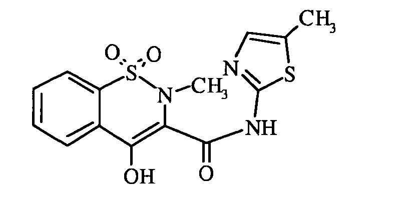 or a pharmaceutically acceptable salt thereof **(2),** and a pharmaceutically acceptable carrier or excipient.

3. A pharmaceutical formulation according to claim 1 or 2 which is suitable for oral, intravascular, intraperitoneal, subcutaneous, intramuscular or topical administration.

4. A pharmaceutical formulation according to any of claims 1 to 3 wherein the weight ratio of **(1)** to **(2)** ranges from 50:1 to 1 : 300, preferably from 8:1 to 1:80.

5. A pharmaceutical formulation according to any of claims 1 to 4 wherein a single application dose contains 1 to 10,000 milligrams, preferably 10 to 2,000 mg of the combined active ingredients **(1)** and **(2).**

6. A pharmaceutical formulation according to any of claims 1 to 5 wherein the pharmaceutically acceptable carrier or excipient is a carbohydrate.

7. Use of effective amounts of a LTB₄ antagonist (**1**) according to claim 1 or 2 and a cyclooxygenase-2 or combined cox1/coxII inhibitor **(2)** according to claim 1 or 2 in a combined form, or separately, or separately and sequentially, wherein the sequential administration is to be close in time or remote in time, for manufacturing a medicament for the prevention or treatment of a disease or disorder selected from die group consisting of arthritis, including rheumatoid arthritis, spondyloarthropathies, gouty arthritis, osteoarthritis, systemic lupus erythematosus and juvenile arthritis, asthma, hay fever, atopic dermatitis, rhinitis, bronchitis, COPD and cystic fibrosis, psoriasis, sclerodermia, morbus bechterew, sarcoidosis, tumor metastasis,morbus crohn, colitis ulcerosa, IBD, multiple sclerosis, arteriosclerosis, arteritis, myocardial infarction, stroke, coronary heart disease in a patient in need thereof.

8. Use according to claim 7 of a LTB₄ antagonist **(1)** according to claim 1 or 2 and a cyclooxygenase-2 inhibitor or combined coxl/2 inhibitor **(2)** according to claim 1 or 2 in a combined form, or separately or separately and sequentially, wherein the sequential administration is to be close in time or remote in time, for the manufacture of a medicamentation for the prevention or treatment of disease or disorder selected from the group consisting of arthritis, including rheumatoid arthritis, spondyloarthropathies, gouty arthritis, osteoarthritis, systemic lupus erythematosus and juvenile arthritis, asthma, bronchitis, COPD and cystic fibrosis.

9. Use according to claim 7 for the manufacture of a medicamentation for the prevention or treatment of rheumatoid arthritis, atopic dermatitis and coronary heart disease.

10. A pharmaceutical kit comprising at least two separate unit dosage forms (A) and (B):
(A) one of which comprises a composition containing LTB₄ antagonist **(1)** according to claim 1 or 2, and optionally a pharmaceutically acceptable carrier;
(B) one of which comprises a composition containing a cyclooxygenase-2 inhibitor or combined coxl/2 inhibitor **(2)** according to claim 1 or 2, and optionally a pharmaceutically acceptable carrier.

## Patentansprüche

1. Pharmazeutische Zusammensetzung, umfassend einen LTB₄-Antagonisten der Formel (I) 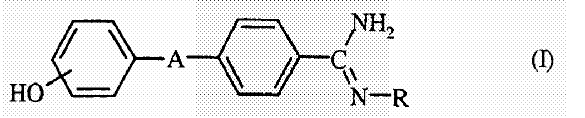 worin R ein Wasserstoffatom oder eine Gruppe der Formel -CO₂-R' darstellt, wobei R' eine C₁₋₆-Alkyl-, eine gegebenenfalls substituierte Phenyl- oder eine gegebenenfalls substituierte Benzyl-Gruppe darstellt, worin die optionalen Substituenten ausgewählt sind aus Halogenatomen, C₁₋₆-Alkyl-, C₁₋₆-Alkoxy-, Cyano-, Nitro-, C₁₋₆-Halogenalkyl- und C₁₋₆-Halogenalkoxy-Gruppen, und A eine Gruppe darstellt, ausgewählt aus der Formel (A1): 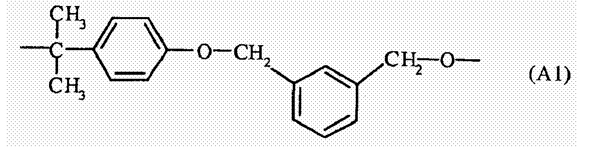 oder einem Tautomer, einem pharmazeutisch akzeptablen bzw. annehmbaren Salz oder Solvat hiervon **(1)** und Meloxicam der Formel 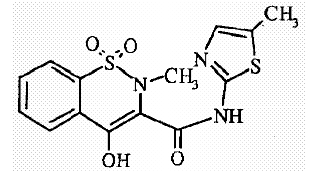 oder einem pharmazeutisch akzeptablen oder annehmbaren Salz hiervon **(2)** und einem pharmazeutisch akzeptablen oder annehmbaren Träger oder Hilfsstoff.

2. Pharmazeutische Zusammensetzung nach Anspruch 1, bestehend im Wesentlichen aus der Verbindung der Formel (IA) 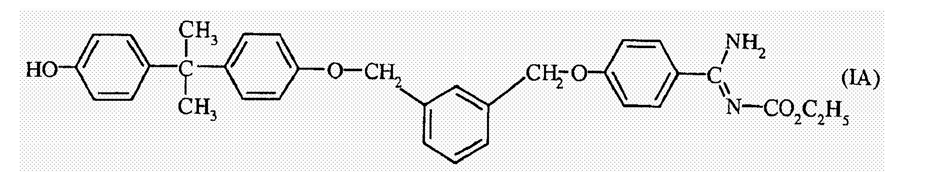 **(1)** und Meloxicam der Formel 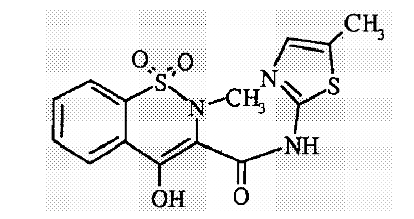 oder einem pharmazeutisch akzeptablen oder annehmbaren Salz hiervon (**2**) und einem pharmazeutisch akzeptablen oder annehmbaren Träger oder Hilfsstoff.

3. Pharmazeutische Formulierung nach Anspruch 1 oder 2, die zur oralen, intravaskulären, intraperitonealen, subkutanen, intramuskulären oder topischen Verabreichung geeignet ist.

4. Pharmazeutische Formulierung nach irgendeinem der Ansprüche 1 bis 3, worin das Gewichtsverhältnis von **(1)** zu **(2)** im Bereich von 50:1 bis 1:300 liegt, bevorzugt von 8:1 bis 1:80.

5. Pharmazeutische Formulierung nach irgendeinem der Ansprüche 1 bis 4, worin eine einzelne Anwendungsdosis 1 bis 10.000 mg, bevorzugt 10 bis 2.000 mg, der kombinierten Wirkstoffe **(1)** und **(2)** enthält.

6. Pharmazeutische Formulierung nach irgendeinem der Ansprüche 1 bis 5, worin der pharmazeutisch akzeptable bzw. annehmbare Träger oder Hilfsstoff ein Kohlenhydrat darstellt.

7. Verwendung wirksamer Mengen eines LTB₄-Antagonisten **(1)** nach Anspruch 1 oder 2 und einem Cyclooxygenase-2- oder einem kombinierten cox 1/cox 2-Inhibitor **(2)** nach Anspruch 1 oder 2 in einer kombinierten Form oder getrennt oder getrennt und hintereinander, worin die sequenzielle Verabreichung kurz hintereinander oder zeitlich getrennt erfolgt, zur Herstellung eines Arzneimittels zur Vorbeugung oder Behandlung einer Krankheit oder Störung, ausgewählt aus der Gruppe, bestehend aus Arthritis, einschließlich rheumatoider Arthritis, Spondyloarthropathien, Gicht (Gauty Arthritis), Osteoarthritis, systemischem Lupus e-rythematodes und Juveniler Arthritis, Asthma, Heuschnupfen, atopischer Dermatitis, Rhinitis, Bronchitis, COPD und zystischer Fibrose, Psoriasis, Sklerodermie, Morbus Bechterew, Sarkoidose, Tumormetastase, Morbus Crohn, ulcerative Colitis, IBD, Multiple Sklerose, Arteriosklerose, Arteritis, Myokardinfarkt, Schlaganfall, koronare Herzerkrankung in einem Patienten mit Bedarf hierfür.

8. Verwendung nach Anspruch 7 eines LTB₄-Antagonisten **(1)** nach Anspruch 1 oder 2 und einem Cyclooxygenase-2- oder einem kombinierten cox 1/cox 2-Inhibitor **(2)** nach Anspruch 1 oder 2 in einer kombinierten Form oder getrennt oder getrennt und hintereinander, worin die sequenzielle Verabreichung kurz hintereinander oder zeitlich getrennt erfolgt, zur Herstellung eines Arzneitmittels zur Vorbeugung oder Behandlung einer Krankheit oder Störung, ausgewählt aus der Gruppe, bestehend aus Arthritis, einschließlich rheumatoider Arthritis, Spondyloarthropathie, Gicht (Gauty Arthritis), Osteoarthritis, systemischem Lupus erythematodes und Juveniler Arthritis, Asthma, Bronchitis, COPD und zystischer Fibrose.

9. Verwendung nach Anspruch 7 zur Herstellung eines Arzneimittels zur Vorbeugung oder Behandlung von rheumatoider Arthritis, atopischer Dermatitis und koronarer Herzerkrankung.

10. Pharmazeutisches Kit, umfassend mindestens zwei getrennte Einheiten von Dosierungsformen (A) und (B):
(A) von denen eine eine Zusammensetzung umfasst, enthaltend den LTB₄-Antagonisten **(1)** nach Anspruch 1 oder 2, und gegebenenfalls einen pharmazeutische akzeptablen bzw. annehmbaren Träger;
(B) von denen eine eine Zusammensetzung umfasst, enthaltend einen Cyclooxygenase-2-Inhibitor oder einen kombinierten cox 1/2-Inhibitor (**2**) nach Anspruch 1 oder 2, und gegebenenfalls einen pharmazeutische akzeptablen bzw. annehmbaren Träger.

## Revendications

1. Composition pharmaceutique comprenant un antagoniste de LTB₄ de formule (I) 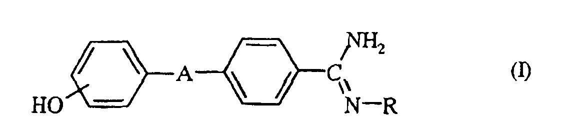 où R représente un atome d'hydrogène ou un groupe de formule -CO₂-R' où R' représente un groupe C₁₋₆ alkyle, un groupe phényle éventuellement substitué ou un groupe benzyle éventuellement substitué, où les substituants éventuels sont choisis parmi les atomes d'halogène, les groupes C₁₋₆ alkyle, C₁₋₆ alcoxy, cyano, nitro ; C₁₋₆ halogénoalkyle et C₁₋₆ halogénoalcoxy, et A est un groupe choisi parmi la formule (A1) : 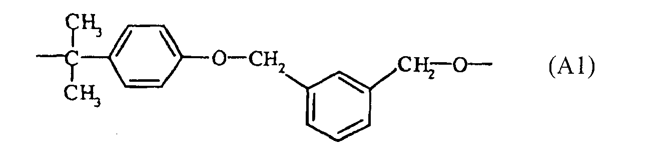 ou un tautomère, un sel pharmaceutiquement acceptable ou un solvate de celui-ci **(1)** et du méloxicam de formule 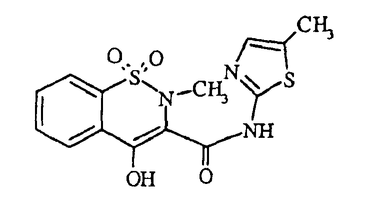 ou un sel pharmaceutiquement acceptable de celui-ci **(2),** et un support ou excipient pharmaceutiquement acceptable.

2. Composition pharmaceutique selon la revendication 1 consistant essentiellement en le composé de formule (IA) 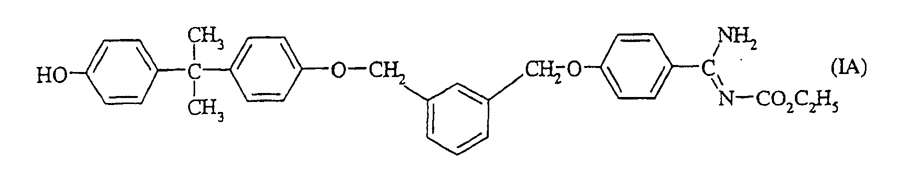 (1) et en méloxicam de formule 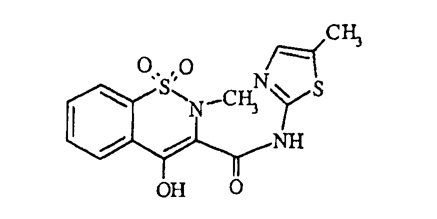 ou un sel pharmaceutiquement acceptable de celui-ci **(2)**, et un support ou excipient pharmaceutiquement acceptable.

3. Formulation pharmaceutique selon la revendication 1 ou 2 qui est appropriée pour l'administration orale, intravasculaire, intrapéritonéale, sous-cutanée, intramusculaire ou topique.

4. Formulation pharmaceutique selon l'une quelconque des revendications 1 à 3 où le rapport massique de **(1)** à **(2)** va de 50 : 1 à 1 : 300, de préférence de 8 : 1 à 1 : 80.

5. Formulation pharmaceutique selon l'une quelconque des revendications 1 à 4 où une dose pour une seule application contient 1 à 10000 milligrammes, de préférence 10 à 2000 mg des ingrédients actifs **(1)** et **(2)** combinés.

6. Formulation pharmaceutique selon l'une quelconque des revendications 1 à 5 où le support ou excipient pharmaceutiquement acceptable est un glucide.

7. Utilisation de quantités efficaces d'un antagoniste de LTB₄ **(1)** selon la revendication 1 ou 2 et d'un inhibiteur de cyclooxygénase-2 ou de coxI/coxII combiné selon la revendication 1 ou 2 dans une forme combinée, ou séparément, ou séparément et successivement, où l'administration successive est destinée à être rapprochée dans le temps ou éloignée dans le temps, pour la fabrication d'un médicament pour la prévention ou le traitement d'une maladie ou affection choisie dans le groupe consistant en l'arthrite, incluant la polyarthrite rhumatoïde, les spondyloarthropathies, l'arthrite goutteuse, l'arthrose, le lupus érythémateux systémique disséminé et l'arthrite juvénile, l'asthme, le rhume des foins, la dermatite atopique, la rhinite, la bronchite, la COPD et la fibrose kystique, le psoriasis, la sclérodermie, la maladie de Bechterew, la sarcoïdose, les métastases tumorales, la maladie de Crohn, la colite ulcéreuse, IBD, la sclérose en plaques, l'artériosclérose, l'artérite, l'infarctus du myocarde, l'attaque, la cardiopathie ischémique, chez un patient qui en a besoin.

8. Utilisation selon la revendication 7 d'un antagoniste de LTB₄ **(1)** selon la revendication 1 ou 2 et d'un inhibiteur de cyclooxygénase-2 ou d'un inhibiteur de cox1/2 combiné **(2)** selon la revendication 1 ou 2 dans une forme combinée, ou séparément, ou séparément et successivement, où l'administration successive est destinée à être rapprochée dans le temps ou éloignée dans le temps, pour la fabrication d'un médicament pour la prévention ou le traitement d'une maladie ou affection choisie dans le groupe consistant en l'arthrite, incluant la polyarthrite rhumatoïde, les spondyloarthropathies, l'arthrite goutteuse, l'arthrose, le lupus érythémateux systémique disséminé et l'arthrite juvénile, l'asthme, la bronchite, la COPD et la fibrose kystique.

9. Utilisation selon la revendication 7 pour la fabrication d'un médicament pour la prévention ou le traitement de la polyarthrite rhumatoïde, de la dermatite atopique et de la cardiopathie ischémique.

10. Kit pharmaceutique comprenant au moins deux formes galéniques unitaires (A) et (B) séparées :
(A) dont l'une comprend une composition contenant un antagoniste de LTB₄ **(1)** selon la revendication 1 ou 2, et éventuellement un support pharmaceutiquement acceptable ;
(B) dont l'une comprend une composition contenant un inhibiteur de cyclooxygénase-2 ou un inhibiteur de cox1/2 combiné **(2)** selon la revendication 1 ou 2, et éventuellement un support pharmaceutiquement acceptable.
